# EUROPÄISCHE PATENTSCHRIFT

(11) **EP 3 586 748 B1**
(45) Veröffentlichungstag und Bekanntmachung des Hinweises auf die Patenterteilung: **24.06.2020**
(21) Anmeldenummer: 18179804.2
(22) Anmeldetag: 26.06.2018
(51) Int. Cl.: A61B 6/00, G06T 7/00, G06T 7/30, A61B 6/03, A61B 8/08, A61B 5/055

(54) **VERFAHREN ZUM BETREIBEN EINES MEDIZINISCHEN BILDGEBUNGSGERÄTS SOWIE BILDGEBUNGSGERÄT**
METHOD FOR OPERATING A MEDICAL IMAGING DEVICE AND IMAGING DEVICE
PROCÉDÉ DE FONCTIONNEMENT D'UN APPAREIL D'IMAGERIE MÉDICALE AINSI QU'APPAREIL D'IMAGERIE

(43) Veröffentlichungstag der Anmeldung: 01.01.2020
(73) Patentinhaber: Siemens Healthcare GmbH, 91052 Erlangen (DE)
(72) Erfinder: Gemmel, Alexander, 91056 Erlangen (DE); Kleinszig, Gerhard, 91301 Forchheim (DE); Kreher, Björn, 91094 Bräuningshof (DE); Kunze, Holger, 91088 Bubenreuth (DE); Magaraggia, Jessica, 91058 Erlangen (DE); Schneider, Stefan, 91058 Erlangen (DE); Weiten, Markus, 90491 Nürnberg (DE)

(56) Entgegenhaltungen:
- EP-A1- 3 316 217
- DE-A1-102015 212 953
- US-A1- 2018 061 059

## Beschreibung

Die Erfindung betrifft ein Verfahren zum Betreiben eines medizinischen Bildgebungsgeräts beim Durchführen einer bildgebenden Untersuchung. Ein zweiter Aspekt der Erfindung betrifft ein medizinisches Bildgebungsgerät.

Beispiele für Bildgebungsgeräte sind Röntgengeräte, Computertomographen und Magnetresonanztomographen. Die bildgebende Untersuchung kann somit eine Röntgenuntersuchung, eine Computertomographie und/oder eine Magnetresonanztomographie umfassen. Derartige bildgebende Untersuchungen werden beispielsweise durchgeführt, um Bilder einer Körperregion eines Patienten zu erzeugen, wie z.B. in der DE 10 2015 212 953 A1 gezeigt. Derartige Bilder können beispielsweise für einen chirurgischen Eingriff herangezogen werden. Die bildgebende Untersuchung ist dabei insbesondere unabhängig von dem chirurgischen Eingriff. Beispielsweise wird die bildgebende Untersuchung vor, nach und/oder in Zwischenphasen des chirurgischen Eingriffs durchgeführt. Das vorliegende Verfahren betrifft insbesondere die technische Auswertung von Rohdaten oder vorverarbeiteten Daten des medizinischen Bildgebungsgeräts, um die genannten Bilder der Körperregion bereit zu stellen.

Beispielsweise kann eine solche bildgebende Untersuchung zum Unterstützen eines chirurgischen Eingriffs zum Behandeln einer Knochenfraktur eingesetzt werden. In diesem Fall können die Bilder mittels des bildgebenden Verfahrens vor, nach oder während dem chirurgischen Eingriff zum Behandeln der Knochenfraktur beziehungsweise eines Knochenbruchs erzeugt werden. Anhand der bildgebenden Untersuchung kann ein behandelnder Arzt, der den chirurgischen Eingriff durchführt, eine räumliche Vorstellung über eine Anatomie der Knochenfraktur und/oder eingebrachter medizinischer Objekte beziehungsweise Implantate erhalten. Eine 3D-Aufnahme während des chirurgischen Eingriffs ist aufwändig, mit einer hohen Strahlenbelastung verbunden und für viele Körperregionen nicht möglich. Insbesondere eine 3D-Röntgenuntersuchung mit einem mobilen Röntgengerät, insbesondere einem C-Bogen, ist zudem mit einem großen Zeitaufwand verbunden. Kontinuierliche 3-D-Aufnahmen sind nicht möglich.

Der Chirurg verschafft sich die räumliche Vorstellung indem er einzelne zweidimensionale Projektionsbilder erfasst. Dabei hängt die Aussagekraft der räumlichen Vorstellung insbesondere davon ab, dass der Arzt jeweilige Projektionsrichtungen für die einzelnen Projektionsbilder geschickt wählt. Durch eine wiederholte Anfertigung solcher Projektionsbilder für unterschiedliche Projektionsrichtungen und Positionen des mobilen Röntgengeräts kann die räumliche Vorstellung ausgebaut beziehungsweise vervollständigt werden. Wie viele Projektionsbilder hierfür nötig sind hängt stark von der Fähigkeit und Erfahrung des Arztes ab. Jedes zusätzliche Projektionsbild führt dabei zu einer Verlängerung einer Operationszeit für den chirurgischen Eingriff und zu einer Erhöhung der Strahlendosis für den Patienten.

Es ist Aufgabe der vorliegenden Erfindung, eine verbesserte Bereitstellung von Bildern im Rahmen einer gattungsgemäßen bildgebenden Untersuchung zu ermöglichen.

Diese Aufgabe wird erfindungsgemäß gelöst durch die Gegenstände der unabhängigen Patentansprüche. Vorteilhafte Ausführungsformen und zweckmäßige Weiterbildungen sind Gegenstand der Unteransprüche.

Ein erster Aspekt der Erfindung geht aus von einem Verfahren zum Betreiben eines medizinischen Bildgebungsgeräts beim Durchführen einer bildgebenden Untersuchung mit den folgenden Verfahrensschritten:
- Bereitstellen eines Ursprungsbildes einer Körperregion,
- Aufnehmen eines Aktualisierungsbildes der Körperregion, und
- Erzeugen eines dreidimensionalen Folgebildes aus dem Ursprungsbild und dem Aktualisierungsbild mittels eines zuvor angelernten künstlichen neuronalen Netzes.

Beim dem Ursprungsbild der Körperregion kann es sich um ein zuvor aufgenommenes Projektionsbild der Körperregion handeln. Somit kann beispielsweise aus dem Ursprungsbild, also dem Projektionsbild, und dem Röntgenbild mittels des künstlichen neuronalen Netzes das dreidimensionale Folgebild erzeugt werden. Beim Bereitstellen des Ursprungsbilds können mehrere Ursprungsbilder bereitgestellt werden, welche im Rahmen des vorliegenden Verfahrens auf dieselbe Weise verarbeitet werden können wie ein einzelnes Ursprungsbild. Insbesondere weisen die mehreren Ursprungsbilder unterschiedliche Projektionsrichtungen in Bezug auf die Körperregion auf.

Bei dem Aktualisierungsbild kann es sich um ein zweidimensionales Projektionsbild handeln. Bei dem Aktualisierungsbild handelt es sich insbesondere um ein Röntgenbild, vorteilhafter Weise ein zweidimensionales Röntgenbild. Das Röntgenbild kann mittels eines mobilen Röntgengeräts, insbesondere mittels eines C-Bogens, aufgenommen werden. Alternativ kann das Aktualisierungsbild mittels eines Ultraschallgeräts, eines Magnetresonanztomographen, einer optischen Kamera oder eines beliebigen anderen bildgebenden Geräts aufgenommen werden. Dementsprechend kann es sich bei dem Aktualisierungsbild beispielsweise um ein Ultraschallbild, eine Magnetresonanztomographie oder ein Kamerabild handeln.

Das Aktualisierungsbild wird insbesondere nicht im Rahmen einer Bildsequenz aufgenommen, welche zum Erzeugen einer dreidimensionalen Repräsentation durchgeführt wird. Somit kann es sich bei mehreren Aktualisierungsbildern um beliebige Bilder mit beliebiger Darstellung der Körperregion handeln.

Das Ursprungsbild kann mittels eines anderen bildgebenden Verfahrens beziehungsweise einem anderen Bildgebungsgerät aufgenommen werden, als das Aktualisierungsbild. In diesem Fall kann vorgesehen sein, dass das medizinische Röntgengerät das Ursprungsbild aus einem weiteren medizinischen Bildgebungsgerät empfängt und anschließend zur weiteren Verarbeitung bereitstellt. Die mit unterschiedlichen bildgebenden Verfahren beziehungsweise unterschiedlichen Bildgebungsgeräten aufgenommenen Bilder, also das Ursprungsbild und das Aktualisierungsbild, können dann zu dem dreidimensionalen Folgebild weiterverarbeitet werden. Mit anderen Worten wird das dreidimensionale Folgebild aus den genannten Bildern erzeugt. Es ist jedoch auch möglich, dass das Ursprungsbild und das Aktualisierungsbild mittels desselben bildgebenden Verfahrens und/oder mittels desselben Bildgebungsgeräts aufgenommen werden. Dementsprechend kann das Ursprungsbild mittels eines mobilen Röntgengeräts, insbesondere eines C-Bogens, aufgenommen werden. In diesem Fall werden insbesondere mehrere Ursprungsbilder aufgenommen, damit durch diese bereits eine räumliche Information betreffend die Körperregion bereitgestellt wird.

Bei der Körperregion kann es sich beispielsweise um einen Teil des (menschlichen) Skeletts handeln. Beispielsweise ist die Körperregion ein einzelner Knochen, eine Mehrzahl an Knochen oder ein Gelenk. Alternativ kann es sich bei der Körperregion um Weichteile des (menschlichen) Körpers handeln, beispielsweise um Lunge, Leber, Gefäße Verdauungstrakt. Im Falle von Weichteilen kann die Ausbreitung eines Kontrastmittels durch das Verfahren visualisiert werden. Zusammengefasst können mittels des vorliegenden Verfahrens beispielsweise Weichteile, Knochen oder Gelenke untersucht werden. Doch auch beliebige andere Körperregionen sind denkbar. Außerdem ist das Verfahren nicht auf den menschlichen Körper beschränkt.

Es kann vorgesehen sein, dass in dem Verfahrensschritt des Aufnehmens des Aktualisierungsbildes mehrere Aktualisierungsbilder aufgenommen werden. Die mehreren Aktualisierungsbilder werden insbesondere alle auf dieselbe Art und Weise bearbeitet, wie das Aktualisierungsbild. Insbesondere weisen die mehreren Aktualisierungsbilder unterschiedliche Projektionsrichtungen in Bezug auf die Körperregion auf.

Das künstliche neuronale Netz kann derart angelernt werden, dass durch das künstliche neuronale Netz aus dem Ursprungsbild und dem Aktualisierungsbild das dreidimensionale Folgebild erzeugen werden können. Umso umfangreicher das künstliche neuronale Netz angelernt ist, desto genauer kann das dreidimensionale Folgebild erzeugt werden. Insbesondere sind zum Erzeugen des dreidimensionalen Folgebilds umso weniger Ursprungsbilder beziehungsweise Aktualisierungsbilder nötig, je vollständiger beziehungsweise umfangreicher das künstliche neuronale Netz angelernt ist. Beim Anlernen des künstlichen neuronalen Netzes kann dieses mit Vorwissen betreffend dem Erzeugen einer dreidimensionalen Repräsentation einer Anatomie anhand von Aktualisierungsbildern und/oder Ursprungsbildern ausgestattet werden. Dieses Vorwissen kann durch das künstliche neuronale Netz zum Erzeugen des dreidimensionalen Folgebilds eingesetzt werden. Bei dem dreidimensionalen Folgebild handelt es sich insbesondere um eine dreidimensionale Repräsentation der Körperregion. Insbesondere wird oder ist das künstliche neuronale Netz darauf angelernt, eines oder mehrere Aktualisierungsbilder in Verbindung mit einem Ursprungbild zum Erzeugen des dreidimensionalen Folgebildes zu nutzen, wobei das dreidimensionale Folgebild und das eine oder die mehreren Aktualisierungsbilder in keinem vorgegebenen Bezug zueinander stehen. Im Falle mehrerer Aktualisierungsbilder kann das künstliche neuronale Netz dazu angelernt werden oder sein, aus dem Ursprungbild und den mehreren Aktualisierungsbildern das dreidimensionale Folgebild zu erzeugen, wobei die mehreren Aktualisierungsbildern in keinem vorgegebenen Bezug zueinander stehen. In keinem vorgegebenen Bezug zueinander bedeutet insbesondere, dass die jeweiligen Bilder wie oben beschrieben nicht Teil einer vorgegebenen Bildsequenz zum Erzeugen einer dreidimensionalen Repräsentation sind. Mit anderen Worten ist oder wird das künstliche neuronale Netz vorteilhafterweise dazu angelernt, Ursprungbild(er) und Aktualisierungsbild(er) der Körperregion, die in beliebigen Verhältnis zueinander stehen, zum Erzeugen des dreidimensionalen Folgebildes zu nutzen.

Es kann vorteilhaft sein, Aufnahmeparameter des Aktualisierungsbildes zur Erzeugung des Folgebildes zu verwenden. Beispielsweise handelt es sich bei dem Aktualisierungsbild um ein Projektionsbild, insbesondere Röntgenbild, welches mittels eines mobilen Röntgengeräts, insbesondere C-Bogen Röntgengeräts, aufgenommen wird. In diesem Beispiel können die Aufnahmeparameter einen oder mehrere der folgenden umfassen: Aufnahmerichtung (Angular, Orbital und die C-Bogen Orientierung), C-Bogen Position, Beschleunigungsspannung, Stromstärke (insbesondere Röhrenstrom), Ladungsmenge (insbesondere Produkt aus Stromstärke und Belichtungsdauer) sowie Strahlengeometrie. Die Aufnahmeparameter können durch das künstliche neuronale Netz zum Erzeugen des dreidimensionalen Folgebildes herangezogen werden. Insbesondere kann das künstliche neuronale Netz dazu angelernt sein beziehungsweise entsprechendes Vorwissen aufweisen, um die Aufnahmeparameter zum verbesserten Erzeugen des dreidimensionalen Folgebildes heranzuziehen.

Gemäß einer Weiterbildung ist vorgesehen, dass ein dreidimensionales Ursprungsbild der Körperregion als das Ursprungsbild bereitgestellt wird. Bei dem dreidimensionalen Ursprungsbild handelt es sich insbesondere um eine dreidimensionale Repräsentation der Körperregion. Bei dem Aktualisierungsbild kann es sich wie oben beschrieben um ein zweidimensionales Aktualisierungsbild, vorteilhafterweise ein zweidimensionales Röntgenbild handeln. Es ist somit vorgesehen, dass beim Erzeugen des dreidimensionalen Folgebilds das dreidimensionale Folgebild aus dem dreidimensionalen Ursprungsbild und dem zweidimensionalen Aktualisierungsbild, insbesondere dem Röntgenbild, erzeugt wird. Mit anderen Worten werden gemäß dieser Ausführungsform zum Erzeugen des dreidimensionalen Folgebilds einerseits ein dreidimensionales Ursprungsbild und andererseits ein zweidimensionales Röntgenbild herangezogen beziehungsweise gemeinsam verarbeitet. Auf diese Weise ist ermöglicht, dass mittels des künstlichen neuronalen Netzes unterschiedlich dimensionale Bilder (zweidimensional und dreidimensional) miteinander verarbeitet werden.

Gemäß einer Weiterbildung ist vorgesehen, dass das Röntgenbild die Körperregion zu einem späteren Zeitpunkt repräsentiert als das Ursprungsbild. Mit anderen Worten kann das Aktualisierungsbild aktueller sein als das Ursprungsbild. Auf diese Weise können ein Informationsgehalt des Ursprungsbilds und des Aktualisierungsbilds besonders vorteilhaft miteinander vermischt werden. Beispielsweise können durch das künstliche neuronale Netz Informationen betreffend einen räumlichen Aufbau der Körperregion aus dem Ursprungsbild mit aktuellen Positionsinformationen, beispielsweise für einen Knochen oder einem medizinischen Objekt, aus dem Aktualisierungsbild fusioniert werden. Auf diese Weise kann das dreidimensionale Folgebild besonders vorteilhaft basierend auf einem oder wenigen Aktualisierungsbildern erzeugt werden.

Gemäß einer Weiterbildung ist vorgesehen, dass das Ursprungsbild im Rahmen einer Computertomographie erzeugt wird und das Aufnehmen des Röntgenbildes mittels eines mobilen Röntgengeräts, insbesondere mittels eines C-Bogen-Röntgengeräts, erfolgt. Mit anderen Worten handelt es sich in diesem Fall bei dem Aktualisierungsbild um ein Röntgenbild. Bei dem Ursprungsbild handelt es sich im vorliegenden Beispiel um eine dreidimensionale Repräsentation beziehungsweise eine dreidimensionale Rekonstruktion der Körperregion. Die Computertomographie kann beispielsweise die Körperstelle vor einem chirurgischen Eingriff charakterisieren. Demgegenüber kann das Aktualisierungsbild die Körperregion während dem chirurgischen Eingriff beziehungsweise während einer Zwischenphase des chirurgischen Eingriffs charakterisieren. Es kann somit das Ursprungsbild, welches die Körperregion vor dem chirurgischen Eingriff charakterisiert, mit dem Aktualisierungsbild, welches die Körperregion während des chirurgischen Eingriffs charakterisiert, zum Erzeugen des dreidimensionalen Folgebilds fusioniert werden. Auf diese Weise können einerseits umfangreiche frühere dreidimensionale Informationen mit aktuellen zweidimensionalen Aktualisierungsbildern, insbesondere Röntgenbildern, fusioniert werden. Hierdurch kann durch das Auswerten des dreidimensionalen Ursprungsbild der Bedarf an aktuellen Aktualisierungsbildern zum Erzeugen des dreidimensionalen Folgebild als aktueller dreidimensionaler Repräsentation der Körperregion verringert werden.

Gemäß einer Weiterbildung ist vorgesehen, dass bei dem Erzeugen des dreidimensionalen Folgebildes das, beispielsweise dreidimensionale, Ursprungsbild der Körperregion anhand des Röntgenbildes zumindest teilweise aktualisiert wird. Beispielsweise wird eine dreidimensionale Repräsentation, welche durch das Ursprungsbild bereitgestellt ist, anhand des aktuelleren Aktualisierungsbilds zumindest teilweise aktualisiert. Dabei können basierend auf dem Vorwissen des zuvor angelernten künstlichen neuronalen Netzes die aktuellen Bildinformationen mit den umfangreichen dreidimensionalen Informationen des Ursprungsbilds fusioniert werden. Das dreidimensionale Folgebild beziehungsweise die durch das dreidimensionale Folgebild bereitgestellte dreidimensionale Repräsentation der Körperregion kann anhand des Aktualisierungsbilds beziehungsweise anhand mehrerer Aktualisierungsbilder aktualisiert werden. Beispielsweise werden strukturelle Änderungen gegenüber dem Ursprungsbild anhand des Aktualisierungsbilds erkannt und das Ursprungsbild zum Erzeugen des dreidimensionalen Folgebilds dementsprechend angepasst. Auf diese Weise kann auch mit wenigen Aktualisierungsbildern eine zuverlässige und aktuelle dreidimensionale Repräsentation in Form des dreidimensionalen Folgebilds bereitgestellt werden.

Insbesondere ist vorgesehen, dass das Ursprungsbild und das Aktualisierungsbild die Körperregion während unterschiedlicher Phasen desselben chirurgischen Eingriffs charakterisieren. Beispielsweise ist vorgesehen, dass das Ursprungsbild während einer vorherigen Phase des chirurgischen Eingriffs verglichen mit dem Aktualisierungsbild aufgenommen wurde. Beispielsweise kann das Ursprungsbild im Rahmen einer Voruntersuchung vor Beginn des chirurgischen Eingriffs erfasst werden. Anschließend kann das Aktualisierungsbild während des chirurgischen Eingriffs aufgenommen werden. Beispiele für unterschiedliche Phasen eines chirurgischen Eingriffs zum Behandeln einer Knochenfraktur sind: Voruntersuchung zum Beurteilen eines Schadens und Auswählen eines Zugangs zur Knochenfraktur, Zusammensetzen der Knochenfraktur, Klammern von zusammengesetzten Knochenfragmenten und abschließendes Fixieren des Knochenfragmente mittels Schrauben und/oder Locheisen. Es ist somit in einem konkreten Beispiel möglich, dass das Ursprungsbild im Rahmen der Voruntersuchung erzeugt wird und die Aktualisierungsbilder während des Zusammensetzen, des Klammerns und/oder des Fixierens aufgenommen werden. Beispielsweise werden mehrere Aktualisierungsbilder während unterschiedlichen der beispielhaft genannten Phasen aufgenommen. Auf diese Weise können Bilder vorausgegangener Phasen eines chirurgischen Eingriffs dazu genutzt werden, das Folgebild der Körperregion für eine spätere Phase des chirurgischen Eingriffs zu erzeugen beziehungsweise bereitzustellen. Das künstliche neuronale Netz ist in diesem Fall dazu angelernt, derartige Aktualisierungsbilder zum Erzeugen des Folgebildes zu nutzen.

Gemäß einer Weiterbildung ist vorgesehen, dass das künstliche neuronale Netz spezifisch auf eine der unterschiedlichen Phasen angelernt wird. Beispielsweise ist für mehrere der unterschiedlichen Phasen ein jeweiliges angelerntes künstliches neuronales Netz vorgesehen. Beispielsweise ist ein künstliches neuronales Netz, welches spezifisch auf die Phase des Zusammensetzens angelernt ist, vorgesehen. Beispielsweise ist ein anderes künstliches neuronales Netz, welches spezifisch auf die Phase des Klammerns und/oder Fixierens angelernt ist, vorgesehen. Beispielsweise kann in einem zusätzlichen Verfahrensschritt aus mehreren künstlichen neuronalen Netzen dasjenige künstliche neuronale Netz, welches auf die aktuell vorliegende Phase angelernt ist, ausgewählt werden. Auf diese Weise kann ein jeweiliges Vorwissen, welches durch Anlernen des künstlichen neuronalen Netzes generiert wird, besonders gut an die jeweilige Phase angepasst sein.

Gemäß einer Weiterbildung ist vorgesehen, dass eine strukturelle Veränderung an der Körperregion, welche zwischen dem Ursprungsbild und dem Aktualisierungsbild aufgetreten ist, bestimmt wird und bei dem Erzeugen des dreidimensionalen Folgebildes berücksichtigt wird. Beispielsweise wird gezielt bestimmt, was sich in dem Aktualisierungsbild verglichen mit dem Ursprungsbild verändert hat. Dies kann dann beim Aktualisieren des Ursprungsbilds anhand des Aktualisierungsbilds berücksichtigt werden. Insbesondere kann die strukturelle Veränderung durch Verschieben von Bildanteilen in dem Ursprungsbild nachgestellt werden. Beispielsweise kann dies nach Art einer Bewegungskompensation für eine Bewegung, welche zwischen dem Ursprungsbild und dem Aktualisierungsbild an der Körperregion aufgetreten ist, durchgeführt werden.

Gemäß einer Weiterbildung ist vorgesehen, dass eine Positionsveränderung eines Knochenfragmentes und/oder eines medizinischen Objekts an der Körperregion als die strukturelle Veränderung bestimmt wird. Beispiele für medizinische Objekte sind Klammern, Locheisen und Schrauben zum Fixieren von Knochen beziehungsweise Knochenfragmenten in der Körperregion. Knochenfragmente und/oder medizinische Objekte können im Rahmen des chirurgischen Eingriffs bewegt werden und/oder an der Körperregion angeordnet werden. Daraus kann eine genannte Positionsveränderung resultieren. Diese Positionsveränderung wird im Rahmen des vorliegenden Verfahrens bestimmt. Ein Verschieben von Knochenfragmenten und/oder Einbringen von medizinischen Objekten ist hierbei ausdrücklich nicht Teil des beanspruchten Verfahrens. Die unterschiedlichen Phasen des chirurgischen Eingriffes, deren Durchführung sowie der chirurgischen Eingriff selbst sind ausdrücklich nicht Teil des beanspruchten Verfahrens. Die Positionsveränderung des Knochenfragments und/oder des medizinischen Objekts kann durch das künstliche neuronale Netz zum Erzeugen des dreidimensionalen Folgebilds anhand des Ursprungsbilds berücksichtigt werden. Insbesondere wird die Positionsveränderung zum Aktualisieren des dreidimensionalen Ursprungsbilds mittels des künstlichen neuronalen Netzes berücksichtigt.

Gemäß einer Weiterbildung ist vorgesehen, dass zur Berücksichtigung der strukturellen Veränderung die Positionsveränderung des Knochenfragmentes und/oder des medizinischen Objekts bestimmt wird und durch Verschieben einer Repräsentation des Knochenfragmentes beziehungsweise des medizinischen Objekts in dem Ursprungsbild berücksichtigt wird. Mit anderen Worten wird die Positionsveränderung durch Verschieben der Repräsentation des Knochenfragments beziehungsweise des medizinischen Objekts in dem Ursprungsbild berücksichtigt. Es kann also in dem Ursprungsbild die Repräsentation des Knochenfragments beziehungsweise des medizinischen Objekts verschoben werden. Dieses Verschieben kann insbesondere entsprechend der vorbestimmten Positionsveränderung erfolgen. Mit anderen Worten kann die Repräsentation des Knochenfragments beziehungsweise des medizinischen Objekts in dem Ursprungsbild in eine durch das Aktualisierungsbild erfasste aktuelle Position bezüglich der Körperregion verschoben werden. Dies ist insbesondere dann vorteilhaft, wenn es sich bei dem Ursprungsbild um ein dreidimensionales Ursprungsbild handelt. Wenn es sich bei dem Ursprungsbild um ein zweidimensionales Ursprungsbild handelt, so kann das Erzeugen des dreidimensionalen Folgebilds anhand des Ursprungsbilds und des Aktualisierungsbilds erst durch Aktualisieren des ersteren ermöglicht werden, da zum Erzeugen der dreidimensionalen Repräsentation der Körperregion die Position des Knochenfragments und/oder des medizinischen Objekts bezüglich der Körperregion in dem Ursprungsbild und dem Aktualisierungsbild übereinstimmen muss.

Gemäß einer Weiterbildung ist vorgesehen, dass das dreidimensionale Folgebild iterativ erzeugt wird, wobei in einem folgenden Iterationsschritt das dreidimensionale Folgebild als neues Ursprungsbild herangezogen wird und zusammen mit einem neuen Aktualisierungsbild ein neues dreidimensionales Folgebild erzeugt wird. Mit anderen Worten kann vorgesehen sein, dass wiederholt Aktualisierungsbilder aufgenommen werden und aus den jeweiligen Aktualisierungsbildern und einem jeweiligen Ursprungsbild ein jeweiliges dreidimensionales Folgebild erzeugt wird. Dabei dient, insbesondere iterativ, jedes der dreidimensionalen Folgebilder als neues Ursprungsbild zum Erzeugen des jeweiligen nachfolgenden dreidimensionalen Folgebilds. Eine dreidimensionale Information, welche durch das dreidimensionale Folgebild bereitgestellt wird, beziehungsweise eine Genauigkeit des dreidimensionalen Folgebilds können somit iterativ anhand der aufeinanderfolgenden Aktualisierungsbilder verbessert werden.

Gemäß einer Weiterbildung ist vorgesehen, dass mehrere Ursprungsbilder bereitgestellt werden und/oder mehrere Röntgenbilder aufgenommen werden, wobei das dreidimensionale Folgebild aus den mehrere Ursprungsbildern und/oder den mehreren Röntgenbildern erzeugt wird. Mit anderen Worten können mehrere Ursprungsbilder als das Ursprungsbild bereitgestellt werden Alternativ oder zusätzlich können mehrere Aktualisierungsbilder als das Aktualisierungsbild aufgenommen werden. Das dreidimensionale Folgebild kann dann entweder aus mehreren Ursprungsbildern und einem Aktualisierungsbild, aus einem Ursprungsbild und mit mehreren Aktualisierungsbildern oder aus mehreren Ursprungsbildern und mehreren Aktualisierungsbildern erzeugt werden. Beispielsweise werden in einem Iterationsschritt des iterativen Verfahrens mehrere Aktualisierungsbilder als das Aktualisierungsbild aufgenommen und innerhalb des einen Iterationsschrittes daraus das dreidimensionale Folgebild erzeugt. In einem folgenden Iterationsschritt können wiederum mehrere Aktualisierungsbilder aufgenommen werden und daraus das neue dreidimensionale Folgebild erzeugt werden. Im Falle mehrerer Aktualisierungsbilder werden diese vorteilhafter Weise jeweils während derselben Phase des chirurgischen Eingriffs aufgenommen. Auf diese Weise kann ein Verarbeitungsaufwand im Falle mehrerer Aktualisierungsbilder beziehungsweise mehrerer Ursprungsbilder verringert werden.

Gemäß einer Weiterbildung ist vorgesehen, dass das künstliche neuronale Netz zumindest teilweise anhand von Testbildern angelernt wird. Die Testbilder können beispielsweise Aktualisierungsbilder von Knochenfrakturen, insbesondere von früheren bildgebender Untersuchungen (insbesondere Röntgenbilder von früheren Röntgenuntersuchungen), und/oder simulierte Röntgenbilder, die anhand einer dreidimensionalen Repräsentation einer Knochenfraktur erzeugt werden, beinhalten. Zusätzlich können die Testbilder Aktualisierungsbilder, insbesondere Röntgenbilder, von künstlich erzeugten Knochenfrakturen, insbesondere an für die medizinische Untersuchung freigegebenen menschlichen Körpern, umfassen. Beispielsweise werden die künstlichen Knochenfrakturen durch Brechen eines Knochens erzeugt und dieser Vorgang mittels einer Vielzahl von Aktualisierungsbildern erfasst. Dabei können jeweilige Aktualisierungsbilder den Knochen im ungebrochenen Zustand und mit unterschiedlichen Graden (bspw. einfach und mehrfach gebrochen) der Knochenfraktur zeigen.

Zusätzlich können den Testbildern jeweilige Aufnahmeparameter (siehe oben) zugeordnet sein. Auf Basis sowohl der Testbilder als auch der zugehörigen Aufnahmeparameter kann dann das künstliche neuronale Netz angelernt werden. Das künstliche neuronale Netz kann mittels der Testbilder und der zugehörigen Aufnahmeparameter darauf angelernt werden, Aufnahmeparameter des Aktualisierungsbildes zum Erzeugen des dreidimensionalen Folgebildes zu nutzen.

Die Aktualisierungsbilder, insbesondere Röntgenbilder, von früheren bildgebenden Untersuchungen können jeweils die jeweilige Knochenfraktur in unterschiedlichen Stadien, also im unbehandelten Zustand, während Zwischenschritten eines jeweiligen chirurgischen Eingriffs und nach Fertigstellung des jeweiligen chirurgischen Eingriffs darstellen.

Die simulierten Aktualisierungsbilder, insbesondere simulierten Röntgenbilder, können beispielsweise anhand einer dreidimensionalen Repräsentation der Knochenfraktur simuliert beziehungsweise erzeugt werden. Bei der dreidimensionalen Repräsentation der Knochenfraktur kann es sich um einen Computertomographie handeln. Anhand der Computertomographie ist eine zweidimensionale Projektion der jeweiligen simulierten Aktualisierungsbilder besonders einfach und umfangreich möglich. Auf diese Weise lassen sich mit geringem Aufwand besonders große Datenmengen an simulierten Aktualisierungsbildern erzeugen. Die simulierten Aktualisierungsbilder können anhand mehrerer dreidimensionaler Repräsentationen der Knochenfraktur erzeugt werden, wobei die mehreren dreidimensionalen Repräsentationen die Knochenfraktur in unterschiedlichen Stadien, beispielsweise in ungebrochenem Zustand und mit unterschiedlichen Graden der Knochenfraktur zeigen.

Dadurch, dass die Testbilder jeweilige Aktualisierungsbilder von unterschiedlichen Graden der Knochenfraktur sowie eines ungebrochenen beziehungsweise vollständig behandelten Knochens darstellen, kann das künstliche neuronale Netz zusätzlich lernen, auf welche Weise die Knochenfraktur im Rahmen des chirurgischen Eingriffs behandelt wird. Positionsveränderungen von Knochenfragmenten und/oder medizinischen Objekten können auf diese Weise durch das künstliche neuronale Netz besonders gut erfasst und/oder interpretiert werden. Auf diese Weise kann das dreidimensionale Folgebild besonders zuverlässig erzeugt werden. Insgesamt ist durch die unterschiedlichen Methoden zur Bereitstellung von Testbildern gezeigt, wie umfangreiche und aussagekräftige Testdaten zum Anlernen des künstlichen neuronalen Netzes bereitgestellt werden können.

Gemäß einer Weiterbildung ist vorgesehen, dass dass das künstliche neuronale Netz spezifisch auf die zu untersuchende Körperregion angelernt wird. Beispielsweise werden jeweilige künstliche neuronale Netze für unterschiedliche Körperregionen angelernt. Es kann dann in Abhängigkeit von der vorliegend zu untersuchenden Körperregion das für die vorliegende Körperregion angelernte künstliche neuronale Netz ausgewählt werden. Beispielsweise handelt es sich bei der Körperregion um ein Knie, ein Schienenbein, ein Armgelenk, einen Armknochen und eine Schulter. Das künstliche neuronale Netz kann dann spezifisch auf genau eine der beispielhaft genannten Körperregionen angelernt werden. Auf diese Weise kann das Vorwissen zum Erzeugen des dreidimensionalen Folgebilds besonders gut an die zu untersuchende Körperregion angepasst sein.

Ein zweiter Aspekt der Erfindung betrifft ein medizinisches Bildgebungsgerät zum Durchführen einer bildgebenden Untersuchung mit
- einer Bereitstellungseinheit zum Bereitstellen eines Ursprungsbildes einer Körperregion,
- einer Aufnahmeeinheit zum Aufnehmen eines Aktualisierungsbilds der Körperregion, und
- einem bestimmungsgemäß angelernten künstlichen neuronalen Netz zum Erzeugen eines dreidimensionalen Folgebildes aus dem Ursprungsbild und dem Aktualisierungsbild. Das medizinische Bildgebungsgerät ist vorteilhafter Weise dazu eingerichtet, ein Verfahren zum Durchführen einer bildgebenden Untersuchung durchzuführen, welches im Rahmen der vorliegenden Anmeldung beschriebene Merkmale aufweist. Die Bereitstellungseinheit kann eine Speichereinheit zum Speichern des Ursprungsbildes und/oder eines Empfangseinheit zum Empfangen des Ursprungsbilds aufweisen. Beispielsweise ist das medizinische Bildgebungsgerät dazu ausgebildet, das Ursprungsbild mittels der Aufnahmeeinheit aufzunehmen oder das Ursprungsbild aus einem weiteren medizinischen Bildgebungsgerät zu empfangen. Bei dem medizinischen Bildgebungsgerät handelt es sich insbesondere um ein mobiles Röntgengerät, vorteilhafter Weise einen C-Bogen.

Die im Rahmen der vorliegenden Anmeldung offenbarten Merkmale des Verfahrens zum Betreiben eines medizinischen Bildgebungsgeräts bilden somit auch das vorliegende medizinische Bildgebungsgerät weiter. Insbesondere weist das medizinische Bildgebungsgerät jeweilige Mittel auf, die dazu eingerichtet sind, Verfahrensschritte und Merkmale des Verfahrens zu realisieren.

Die vorliegende Erfindung wird nun anhand von mehreren Zeichnungen näher erläutert. Dabei zeigen:
- FIG 1:: eine schematische Darstellung eines medizinischen Bildgebungsgeräts;
- FIG 2:: eine schematische Übersicht über einen zeitlichen Verlauf einer beispielhaften Ausführungsform des vorliegenden Verfahrens; und
- FIG 3:: ein Flussdiagramm des beispielhaften Verfahrens.

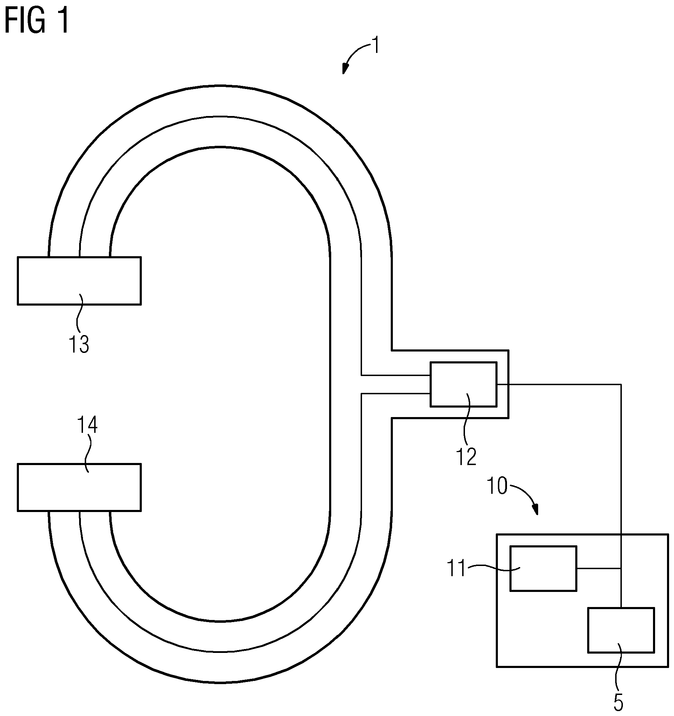

FIG 1 zeigt ein medizinisches Bildgebungsgerät 1, vorliegend ein so genanntes C-Bogen-Röntgengerät. Das Bildgebungsgerät 1 weist vorliegend eine Röntgenquelle 13 sowie einen Detektor 14 auf. Zudem umfasst das Bildgebungsgerät 1 eine Aufnahmeeinheit 12 zum Aufnehmen von Aktualisierungsbildern 3. Bei den Aktualisierungsbildern 3 handelt es sich im vorliegenden Ausführungsbeispiel um Röntgenbilder. Zusätzlich umfasst das Bildgebungsgerät 1 eine Steuereinrichtung 10 mit einer Bereitstellungseinheit 11 und einem künstlichen neuronalen Netz 5. Das künstliche neuronale Netz 5 ist in einem betriebsbereiten Zustand des medizinischen Bildgebungsgeräts 1 bestimmungsgemäß angelernt.

Wird das medizinische Bildgebungsgerät 1 in eine bestimmungsgemäße Relativposition bezogen auf einen zu untersuchenden Patienten gebracht, so kann ein Aktualisierungsbild 3 einer Körperregion des Patienten aufgenommen werden.

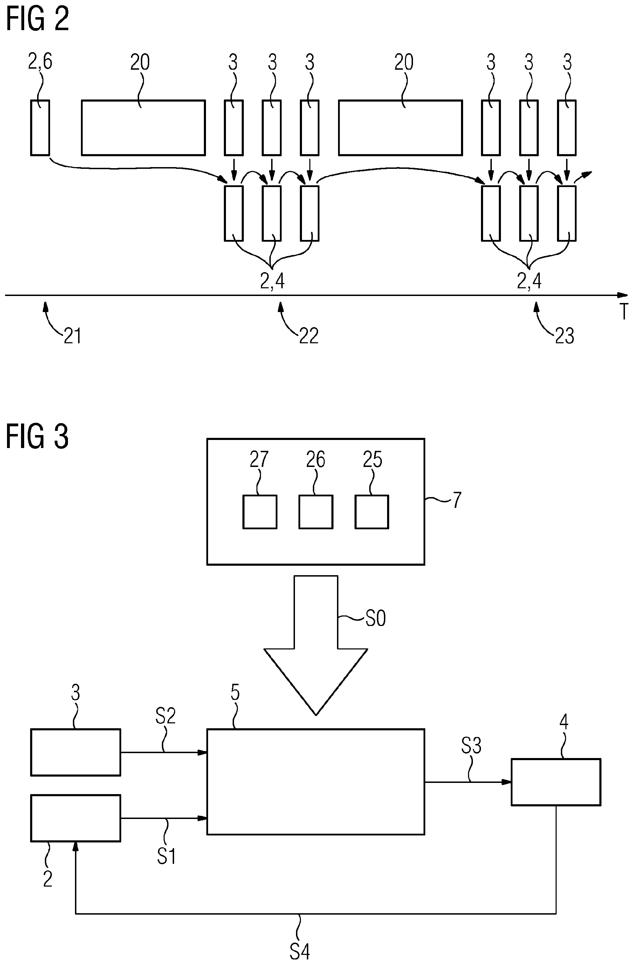

FIG 2 zeigt einen beispielhaften zeitlichen Verlauf eines Verfahrens zum Betreiben des medizinischen Bildgebungsgeräts 1. Jeweilige Aktualisierungsbilder 3 werden während jeweiliger unterschiedlicher Phasen 22, 23, 24 bezogen auf einen chirurgischen Eingriff 20 aufgenommen. Die unterschiedlichen Phasen 22, 23, 24 beziehen sich auf denselben chirurgischen Eingriff 20. Der chirurgischen Eingriff 20 ist auf einer Zeitleiste T ebenfalls dargestellt, jedoch nicht Teil des vorliegenden Verfahrens. Der chirurgische Eingriff 20 kann beispielsweise zum Behandeln einer Knochenfraktur des zu untersuchenden Patienten vorgesehen sein. Ein Zustand der Knochenfraktur und/oder der Behandlung der Knochenfraktur kann mittels einer bildgebenden Untersuchung bestimmt oder überprüft werden. Die Phasen 22, 23, 24 können Zwischenphasen des chirurgischen Eingriffs 20 sein. Die Aktualisierungsbilder 3 werden vorliegend während jeweiliger Zwischenphasen des chirurgischen Eingriffs 20 durch das medizinische Bildgebungsgerät 1 aufgenommen. In einer ersten Phase 21 vor Beginn des eigentlichen chirurgischen Eingriffes 20, einer so genannten Voruntersuchungsphase, wird entweder ein dreidimensionales Ursprungsbild 2 oder werden mehrere zweidimensionale Ursprungsbilder 2 erzeugt beziehungsweise aufgenommen. Zur Abgrenzung von späteren Ursprungsbildern 2, wird das Ursprungsbild 2 beziehungsweise werden die Ursprungsbilder 2 der ersten Phase 21 als erstes Ursprungsbild 6 beziehungsweise erste Ursprungsbilder 6 bezeichnet. Anhand der Ursprungsbilder 2 und der Aktualisierungsbilder 3 wird das vorliegende Verfahren eine verbesserte Übersicht über die Körperregion ermöglicht. Der chirurgische Eingriff 20 ist explizit nicht Teil des vorliegenden Verfahrens.

Im Falle eines dreidimensionalen ersten Ursprungsbilds 6 kann dieses mittels eines Computertomographen aufgenommen werden. In diesem Fall empfängt das Bildgebungsgerät 1 beziehungsweise die Bereitstellungseinheit 11 das erste Ursprungsbild 6 aus dem Computertomographen. Hierbei kann der Empfang auch indirekt, beispielsweise über einen Datenträger zur Zwischenspeicherung, erfolgen. Hierzu kann die Bereitstellungseinheit 11 eine Schnittstelle aufweisen, beispielsweise einen USB-Anschluss oder einen Netzwerkanschluss. Im Falle mehrerer erster Ursprungsbilder 6 können diese mittels des Bildgebungsgeräts 1 aufgenommen werden. Doch auch im Falle mehrerer erster Ursprungsbilder 6 können diese aus einem weiteren Bildgebungsgerät empfangen werden. Vorliegend handelt es sich bei den mehreren Ursprungsbildern 6 um jeweilige zweidimensionale Röntgenbilder, welche mittels der Aufnahmeeinheit 12 aufgenommen werden. Die ersten Ursprungsbilder 6 beziehungsweise das erste Ursprungsbild 6 können durch die Bereitstellungseinheit 11 aus der Aufnahmeeinheit 12 empfangen werden und zwischengespeichert werden. Die erste Phase 21 kann eine Voruntersuchung sein, welche kurz vor Beginn des chirurgischen Eingriffs 20 durchgeführt wird. Mit anderen Worten kann der chirurgischen Eingriff 20 direkt im Anschluss an die erste Phase 21 erfolgen. Die ersten Ursprungsbilder 6 beziehungsweise das erste Ursprungsbild 6 charakterisieren die Körperregion somit zu Beginn des chirurgischen Eingriffs 20.

Im Rahmen der Voruntersuchung beziehungsweise der ersten Phase 21 kann beispielsweise ein geeigneter Zugang für den chirurgischen Eingriff 20 bestimmt werden.

Eine zweite Phase 22 kann den chirurgischen Eingriff 20 zeitlich betrachtet unterbrechen beziehungsweise pausieren. Während der zweiten Phase 22 werden mehrere Aktualisierungsbilder 3, vorliegend Röntgenbilder, der Körperregion mittels der Aufnahmeeinheit 12 aufgenommen. Es wird iterativ aus jedem der Aktualisierungsbilder 3 sowie einem vorherigen Ursprungsbild 2 ein dreidimensionales Folgebild 4 erzeugt. Dabei wird das erzeugte Folgebild 4 jeweils zum Erzeugen des nachfolgenden Folgebilds 4 als Ursprungsbild 2 herangezogen. Anhand der Pfeile in FIG 2 wird dies gut sichtbar. Konkret wird ein erstes der Folgebilder 4 aus dem ersten Ursprungsbild 6 beziehungsweise den ersten Ursprungsbildern 6 sowie einem ersten der Aktualisierungsbilder 3 erzeugt. Ein zweites der Folgebilder 4 wird auch einem zweiten der Aktualisierungsbilder 3 sowie dem ersten der Folgebilder 4 erzeugt. Das erste der Folgebilder 4 wird somit als Ursprungsbild zum Erzeugen für das zweite der Folgebilder 4 herangezogen. Die Angaben "erstes" und "zweites" sind hierbei in Bezug auf die Zeitachse T zu verstehen. Das zweite der Folgebilder 4 charakterisiert die Körperregion somit zu einem späteren Zeitpunkt als das erste der Folgebilder 4.

Dies ist auch in FIG 3 dargestellt. In einem Schritt S1 wird das jeweilige Ursprungsbild 2 für das künstliche neuronale Netz 5 bereitgestellt. Das künstliche neuronale Netz wurde in einem vorherigen Schritt S0 angelernt, was im Folgenden noch genauer erläutert wird. In einem Schritt S2 wird ein jeweiliges Aktualisierungsbild 3 aufgenommen und dem künstlichen neuronalen Netz 5 zugeführt. Das künstliche neuronale Netz 5 erzeugt in einem Schritt S3 das jeweilige Folgebild 4 aus dem Ursprungsbild 2 und dem Aktualisierungsbild 3. In einem Iterationsschritt S4 wird das erzeugte dreidimensionale Folgebild 4 als neues Ursprungsbild 2 herangezogen. Das neue Ursprungsbild 2 kann mit einem neuen Aktualisierungsbild 3 wiederum dem künstlichen neuronalen Netz 5 zum Erzeugen eines neuen Folgebilds 4 zugeführt werden.

Nach der zweiten Phase 22 kann der chirurgischen Eingriff 20 fortgesetzt werden (FIG 2). Nach Beendigung des chirurgischen Eingriffs 20 folgt eine dritte Phase 23. Die Aktualisierungsbilder 3 der dritten Phase 23 charakterisieren die Körperregion somit zu einem Ende des chirurgischen Eingriffs 20. Die Aktualisierungsbilder 3 der zweiten Phase 22 charakterisieren die Körperregion während einer Zwischenphase des chirurgischen Eingriffs 20. Das Erzeugen von dreidimensionalen Folgebildern 4 in der dritten Phase 23 erfolgt analog zum Erzeugen von dreidimensionalen Folgebildern 4 in der zweiten Phase 22. Beim Erzeugen des jeweils ersten Folgebilds 4 einer der Phasen 22, 23 wird das Ursprungsbild 2 aus der jeweils vorherigen Phase 21, 22 herangezogen. Zum Erzeugen des ersten Folgebilds 4 der zweiten Phase 22 wird das erste Ursprungsbild 6 beziehungsweise werden die ersten Ursprungsbilder 6 aus der ersten Phase 21 herangezogen. Zum Erzeugen eines ersten der Folgebilder 4 der zweiten Phase 23 wird als das Ursprungsbild 2 ein Folgebild 4 aus der zweiten Phase 22 herangezogen. Somit wird das jeweilige Folgebild 4 in den genannten Fällen aus jeweils einem Ursprungsbild 2 und jeweils einem Aktualisierungsbild 3 erzeugt, welche die Körperregion während unterschiedlicher Phasen des chirurgischen Eingriffs 20 charakterisieren.

Während dem chirurgischen Eingriff 20 können strukturelle Veränderungen an der Körperregion auftreten. Im Falle einer Knochenfraktur kann eine solche strukturelle Veränderung beispielsweise durch eine Positionsveränderung eines Knochenfragmentes und/oder eines medizinischen Objekts an der Körperregion resultieren. Beispielsweise werden während des chirurgischen Eingriffs 20 Knochenfragmente zum Behandeln der Knochenfraktur relativ zueinander verschoben beziehungsweise aneinander ausgerichtet. Alternativ oder zusätzlich können im Rahmen des chirurgischen Eingriffs 20 medizinische Objekte an der Körperregion angeordnet werden oder verschoben werden. Beispiele für medizinische Objekte sind Klammern, Schrauben und Locheisen zum Fixieren der Knochenfragmente. Bezogen auf die Zeitachse T in FIG 2 kann beispielsweise zwischen der ersten Phase 21 und der zweiten Phase 22 im Rahmen des medizinischen Eingriffs eine Ausrichtung der Knochenfragmente zueinander vorgesehen sein. Hierbei können die Knochenfragmente durch den behandelnden Arzt zusammengesetzt werden. Zwischen der zweiten Phase 22 und der dritten Phase 23 kann das Einbringen der medizinischen Objekte zum Fixieren der Knochenfragmente vorgesehen sein. Das Verschieben der Knochenfragmente und Fixieren derselben ist ausdrücklich nicht Teil des vorliegenden Verfahrens. Das vorliegende Verfahren ermöglicht es jedoch, den Fortschritt des Verschiebens der Knochenfragmente und/oder des Anordnens der medizinischen Objekte visuell für den behandelnden Arzt aufzubereiten. Dabei kann dem behandelnden Arzt in Form der Folgebilder 4 eine räumliche Repräsentation der Körperregion bereitgestellt werden. Bei dem dreidimensionalen Folgebild 4 kann es sich um die die räumliche Repräsentation der Körperregion handeln. Insbesondere werden zum Erstellen des dreidimensionalen Folgebildes 4 Aktualisierungsbilder 3, die in unterschiedlichen Phasen 22, 23, 24 des chirurgischen Eingriffs 20 aufgenommen werden, herangezogen. Mit anderen Worten kann das dreidimensionale Folgebild 4 aus Aktualisierungsbildern 3, die in unterschiedlichen Phasen 22, 23, 24 des chirurgischen Eingriffs 20 aufgenommen werden, gebildet werden.

Um das Erzeugen der dreidimensionalen Folgebilder 4 mittels des künstlichen neuronalen Netzes 5 zu ermöglichen, muss das künstliche neuronale Netz zunächst in einem Schritt S0 angelernt werden. Hierbei wird durch das künstliche neuronale Netz Vorwissen generiert, anhand welchem das künstliche neuronale Netz in der Lage ist, das dreidimensionale Folgebild 4 zu erzeugen. Ohne die Generierung dieses Vorwissens ist das Erzeugen des dreidimensionalen Folgebilds 4 nicht ohne weiteres möglich, da im Falle weniger Aktualisierungsbilder 3 oder stark abweichender Aktualisierungsbilder 3 (aufgrund struktureller Veränderungen im Rahmen des chirurgischen Eingriffs 20) es sich um ein unterbestimmtes System handeln kann. Unterbestimmt heißt hierbei, dass zu wenige unterschiedliche Projektionsrichtungen durch die Aktualisierungsbilder 3 bereitgestellt sind. Diese Unterbestimmtheit des vorliegenden Systems an Aktualisierungsbildern und Ursprungsbildern kann durch das Vorwissen des künstlichen neuronalen Netzes ausgeglichen werden. Das Vorwissen des künstlichen neuronalen Netzes 5 betrifft vorteilhafter Weise typische Strukturen für die Körperregion. Dies basiert auf der Überlegung, dass bestimmte Körperregionen bei einer Vielzahl von Menschen große Ähnlichkeiten aufweisen.

Das Anlernen des künstlichen neuronalen Netzes 5 erfolgt anhand von Trainingsdaten, welche insbesondere Testbilder 7 umfassen. Die Trainingsdaten beziehungsweise Testbilder 7 können beispielsweise simulierte Aktualisierungsbilder 25, Aktualisierungsbilder 26 von früheren chirurgischen Eingriffen beziehungsweise früheren bildgebenden Untersuchungen, sowie Aktualisierungsbilder 27 von künstlich gebrochenen Knochen von Körpern, die für medizinische Untersuchungen beziehungsweise Entwicklungen freigegeben sind, umfassen. Die dreidimensionalen Repräsentationen zum Erzeugen der simulierten Aktualisierungsbilder 25 können beispielsweise durch eine Computertomographie bereitgestellt werden. Beispielsweise wird eine solche Computertomographie an künstlichen Brüchen für die Aktualisierungsbilder 27 oder anhand von früheren bildgebenden Untersuchungen durchgeführt.

Bei den unterschiedlichen Aktualisierungsbildern 25, 26, 27 ist es jeweils vorteilhaft, wenn eine jeweilige Knochenfraktur durch die jeweiligen Aktualisierungsbilder 25, 26, 27 in unterschiedlichen Stadien repräsentiert ist. Die unterschiedlichen Stadien können beispielsweise einen nicht frakturierten Knochen, einen einfach gebrochenen Knochen und einen mehrfach gebrochenen Knochen beziehungsweise unterschiedliche Grade an Dislokation von Knochenfragmenten betreffen. Beispielsweise werden jeweilige Sätze an Aktualisierungsbildern 25, 26, 27 erzeugt, für einen nicht gebrochenen Knochen beziehungsweise zusammengefügten Knochen, einen einfach gebrochenen Knochen, einen mehrfach gebrochenen Knochen und für verschiedene Grade an Dislokation der Knochenfragmente. Auf diese Weise kann das künstliche neuronale Netz 5 auch in Bezug auf einen Verlauf struktureller Veränderungen an der Körperregion im Verlauf des chirurgischen Eingriffs 20 angelernt werden.

Die Trainingsdaten beziehungsweise die Testbilder 7 können spezifisch für eine bestimmte Körperregion sein. Somit kann das künstliche neuronale Netz 5 spezifisch an eine bestimmte Körperregion angelernt werden. Beispiele für Körperregionen, die Trainingsdaten beziehungsweise die Testbilder 7 repräsentiert sein können und an die das künstliche neuronale Netz 5 angelernt werden kann sind: Knie, Schienbein, Armgelenk beziehungsweise Armbeuge, Unterarmknochen sowie Schulter. Diese Aufzählung ist nicht beschränkend zu verstehen. Zum Durchführen der bildgebenden Untersuchung kann ein für die zur Untersuchung der Körperregion spezifisches künstliches neuronales Netz 5 aus einer Mehrzahl an künstlichen neuronalen Netzen ausgewählt werden.

Es ist vorgesehen, dass durch das künstliche neuronale Netz 5 strukturelle Veränderungen an der Körperregion bestimmt werden, welche zwischen dem Ursprungsbild 2 und dem Aktualisierungsbild 3 aufgetreten sind. Zum Erzeugen des dreidimensionalen Folgebilds 4 wird eine solche strukturelle Veränderung berücksichtigt. Diese Berücksichtigung erfolgt insbesondere durch Bestimmen von Positionsveränderungen von Knochenfragmenten und/oder medizinischer Objekte zwischen dem Ursprungsbild 2 und dem Aktualisierungsbild 3. Liegt eine solche Positionsveränderung vor, so wird diese durch Verschieben einer Repräsentation des jeweiligen Knochenfragmentes beziehungsweise des jeweiligen medizinischen Objektes in dem Ursprungsbild 2 berücksichtigt. Mit anderen Worten kann durch das künstliche neuronale Netz 5 die Repräsentation eines Knochenfragmentes beziehungsweise medizinischen Objekts in dem Ursprungsbild verschoben werden, wenn sich eine Position des jeweiligen Knochenfragmentes und/oder des medizinischen Objekts in dem Aktualisierungsbild 3 verändert hat. Dies kann als eine Bewegungskompensation aufgefasst werden, in der eine Bewegung des Knochenfragmentes und/oder des medizinischen Objekts durch Verschiebung der jeweiligen Repräsentation in dem Ursprungsbild 2 kompensiert wird. Auf diese Weise kann eine Position, in der das Knochenfragment beziehungsweise das medizinische Objekt in dem Ursprungsbild 2 dargestellt wird, an das aktuellere Aktualisierungsbild 3 angepasst werden.

Insgesamt ist durch das Ausführungsbeispiel gezeigt, wie in Form des dreidimensionalen Folgebilds eine verbesserte Repräsentation einer Körperregion bereitgestellt werden kann.

## Patentansprüche

1. Verfahren zum Betreiben eines medizinischen Bildgebungsgerätes (1) beim Durchführen einer bildgebenden Untersuchung, mit den Verfahrensschritten:
- Bereitstellen (S1) eines Ursprungsbildes (2) einer Körperregion;
- Aufnehmen eines Aktualisierungsbildes (3) der Körperregion; und
- Erzeugen eines dreidimensionalen Folgebildes (4) aus dem Ursprungsbild (2) und dem Aktualisierungsbild (3) mittels eines zuvor angelernten künstlichen neuronalen Netzes (5).

2. Verfahren nach Anspruch 1, **dadurch gekennzeichnet, dass** ein dreidimensionales Ursprungsbild (2) der Körperregion als das Ursprungsbild (2) bereitgestellt wird.

3. Verfahren nach einem der vorhergehenden Ansprüche, **dadurch gekennzeichnet, dass** das Aktualisierungsbild (3) die Körperregion zu einem späteren Zeitpunkt repräsentiert als das Ursprungsbild (2).

4. Verfahren nach einem der vorhergehenden Ansprüche, **dadurch gekennzeichnet, dass** das Ursprungsbild (2) im Rahmen einer Computertomographie erzeugt wird und das Aufnehmen des Aktualisierungsbildes (3) mittels eines mobilen Röntgengeräts, insbesondere mittels eines C-Bogen-Röntgengeräts, erfolgt.

5. Verfahren nach einem der vorhergehenden Ansprüche, **dadurch gekennzeichnet, dass** bei dem Erzeugen des dreidimensionalen Folgebildes (4) das Ursprungsbild (2) der Körperregion anhand des Aktualisierungsbildes (3) zumindest teilweise aktualisiert wird.

6. Verfahren nach einem der vorhergehenden Ansprüche, **dadurch gekennzeichnet, dass** das Ursprungsbild (2) und das Aktualisierungsbild (3) die Körperregion während unterschiedlicher Phasen (21, 22, 23) eines gleichen chirurgischen Eingriffs (20) charakterisieren.

7. Verfahren nach den Anspruch 6, **dadurch gekennzeichnet, dass** das künstliche neuronale Netz (5) spezifisch auf eine der unterschiedlichen Phasen (21, 22, 23)angelernt wird.

8. Verfahren nach einem der vorhergehenden Ansprüche, **dadurch gekennzeichnet, dass** eine strukturelle Veränderung an der Körperregion, welche zwischen dem Ursprungsbild (2) und dem Aktualisierungsbild (3) aufgetreten ist, bestimmt wird und bei dem Erzeugen des dreidimensionalen Folgebildes (4) berücksichtigt wird.

9. Verfahren nach Anspruch 8, **dadurch gekennzeichnet, dass** eine Positionsveränderung eines Knochenfragmentes und/oder eines medizinischen Objekts an der Körperregion als die strukturelle Veränderung bestimmt wird.

10. Verfahren nach Anspruch 9, **dadurch gekennzeichnet, dass** zur Berücksichtigung der strukturellen Veränderung die Positionsveränderung des Knochenfragmentes und/oder des medizinischen Objekts bestimmt wird und durch Verschieben einer Repräsentation des Knochenfragmentes beziehungsweise des medizinischen Objekts in dem Ursprungsbild berücksichtigt wird.

11. Verfahren nach einem der vorhergehenden Ansprüche, **dadurch gekennzeichnet, dass** das dreidimensionale Folgebild (4) iterativ erzeugt, wobei in einem folgenden Iterationsschritt (S4) das dreidimensionale Folgebild (4) als neues Ursprungsbild (2) herangezogen wird und zusammen mit einem neuen Aktualisierungsbild (3) ein neues dreidimensionales Folgebild (4) erzeugt wird.

12. Verfahren nach einem der vorhergehenden Ansprüche, **dadurch gekennzeichnet, dass** mehrere Ursprungsbilder (2) bereitgestellt werden und/oder mehrere Aktualisierungsbilder (3) aufgenommen werden, wobei das dreidimensionale Folgebild (4) aus den mehrere Ursprungsbildern (2) und/oder den mehreren Aktualisierungsbildern (3) erzeugt wird.

13. Verfahren nach einem der vorhergehenden Ansprüche, **dadurch gekennzeichnet, dass** das künstliche neuronale Netz (5) zumindest teilweise anhand von Testbildern (7) angelernt wird, wobei die Testbilder (7) Aktualisierungsbilder (26) von Knochenfrakturen, insbesondere von früheren bildgebenden Untersuchungen, und/oder simulierte Aktualisierungsbilder (25), die anhand einer dreidimensionalen Repräsentation einer Knochenfraktur erzeugt werden, beinhalten.

14. Verfahren nach einem der vorhergehenden Ansprüche, **dadurch gekennzeichnet, dass** das künstliche neuronale Netz (5) spezifisch auf die zu untersuchende Körperregion angelernt wird.

15. Medizinisches Bildgebungsgerät (1) zum Durchführen einer bildgebenden Untersuchung, mit
- einer Bereitstellungseinheit (20) zum Bereitstellen eines Ursprungsbildes (2) einer Körperregion;
- einer Aufnahmeeinheit (21) zum Aufnehmen eines Aktualisierungsbildes (3) der Körperregion; und
- einem bestimmungsgemäß angelernten künstlichen neuronalen Netz(5) zum Erzeugen eines dreidimensionalen Folgebildes (4) aus dem Ursprungsbild (2) und dem Aktualisierungsbild (3) .

## Claims

1. Method for operating a medical imaging device (1) when performing an imaging examination, with the process steps:
- providing (S1) an original image (2) of a body region,
- recording an updated image (3) of the body region, and
- generating a three-dimensional subsequent image (4) from the original image (2) and from the updated image (3) using a previously trained artificial neural network (5).

2. Method according to claim 1, **characterised in that** a three-dimensional original image (2) of the body region is provided as the original image (2).

3. Method according to one of the preceding claims, **characterised in that** the updated image (3) represents the body region at a later time than the original image (2).

4. Method according to one of the preceding claims, **characterised in that** the original image (2) is generated in the context of computed tomography and the recording of the updated image (3) ensues using a mobile X-ray device, in particular using a C-arm X-ray device.

5. Method according to one of the preceding claims, **characterised in that**, in the generation of the three-dimensional subsequent image (4), the original image (2) of the body region is at least partly updated using the updated image (3).

6. Method according to one of the preceding claims, **characterised in that** the original image (2) and the updated image (3) characterise the body region during different phases (21, 22, 23) of the same surgical intervention (20).

7. Method according to claim 6, **characterised in that** the artificial neural network (5) is trained specifically in one of the different phases (21, 22, 23).

8. Method according to one of the preceding claims, **characterised in that** a structural change in the body region, which has occurred between the original image (2) and the updated image (3), is determined and accounted for in the generation of the three-dimensional subsequent image (4).

9. Method according to claim 8, **characterised in that** a change in position of a bone fragment and/or of a medical object is determined in the body region as the structural change.

10. Method according to claim 9, **characterised in that** to account for the structural change, the change in position of the bone fragment and/or of the medical object is determined and is accounted for by moving a representation of the bone fragment or of the medical object in the original image.

11. Method according to one of the preceding claims, **characterised in that** the three-dimensional subsequent image (4) is generated iteratively, wherein in a subsequent iteration step (S4), the three-dimensional subsequent image (4) is used as a new original image (2) and a new three-dimensional subsequent image (4) is generated together with a new updated image (3).

12. Method according to one of the preceding claims, **characterised in that** a plurality of original images (2) are provided and/or a plurality of updated images (3) are recorded, wherein the three-dimensional subsequent image (4) is generated from the plurality of original images (2) and/or the plurality of updated images (3).

13. Method according to one of the preceding claims, **characterised in that** the artificial neural network (5) is at least partly trained using test images (7), wherein the test images (7) contain updated images (26) of bone fractures, in particular from earlier imaging examinations, and/or simulated updated images (25), which are generated using a three-dimensional representation of a bone fracture.

14. Method according to one of the preceding claims, **characterised in that** the artificial neural network (5) is trained specifically in the body region that is to be examined.

15. Medical imaging device (1) for performing an imaging examination, comprising
- a providing unit (20) to prepare an original image (2) of a body region;
- an imaging unit (21) to record an updated image (3) of the body region; and
- an artificial neural network (5) trained according to the intended use to generate a three-dimensional subsequent image (4) from the original image (2) and from the updated image (3) .

## Revendications

1. Procédé pour faire fonctionner un appareil (1) d'imagerie médicale lorsque l'on effectue un examen par imagerie, comprenant les stades de procédé :
- on se procure (S1) une image (2) d'origine d'une région du corps ;
- on enregistre une image (3) mise à jour de la région du corps ; et
- on produit une image (4) de suite en trois dimensions à partir de l'image (2) d'origine et de l'image (3) mise à jour au moyen d'un réseau (3) neuronal artificiel ayant subi un apprentissage auparavant.

2. Procédé suivant la revendication 1, **caractérisé en ce que** l'on se procure une image (2) d'origine en trois dimensions de la région du corps, comme image (2) d'origine.

3. Procédé suivant l'une des revendications précédentes, **caractérisé en ce que** l'image (3) mise à jour représente la région du corps à un instant plus tardif que ne le fait l'image (2) d'origine.

4. Procédé suivant l'une des revendications précédentes, **caractérisé en ce que** l'on produit l'image (2) d'origine dans le cadre d'une tomographie par ordinateur et **en ce que** l'enregistrement de l'image (3) mise à jour s'effectue au moyen d'un appareil aux rayons X mobile, notamment au moyen d'un appareil aux rayons X à arceau en C.

5. Procédé suivant l'une des revendications précédentes, **caractérisé en ce que**, lors de la production de l'image (4) de suite en trois dimensions, on met à jour, au moins en partie, l'image (2) d'origine de la région du corps à l'aide de l'image (3) de mise à jour.

6. Procédé suivant l'une des revendications précédentes, **caractérisé en ce que** l'image (2) d'origine et l'image (3) mise à jour caractérisent la région du corps pendant des phases (21, 22, 23) différentes d'une même intervention (20) chirurgicale.

7. Procédé suivant la revendication 6, **caractérisé en ce que** l'on fait subir au réseau (5) neuronal artificiel un apprentissage spécifique à l'une des phases (21, 22, 23) différentes.

8. Procédé suivant l'une des revendications précédentes, **caractérisé en ce que** l'on détermine une modification structurelle de la région du corps, qui se produit entre l'image (2) d'origine et l'image (3) de mise à jour et on en tient compte lors de la production de l'image (4) de suite en trois dimensions.

9. Procédé suivant la revendication 8, **caractérisé en ce que** l'on détermine une modification de position d'un fragment d'os et/ou d'un objet médical dans la région du corps comme modification structurelle.

10. Procédé suivant la revendication 9, **caractérisé en ce que**, pour tenir compte de la modification structurelle, on détermine la modification de position du fragment d'os et/ou de l'objet médical et on en tient compte par déplacement d'une représentation du fragment d'os ou de l'objet médical dans l'image d'origine.

11. Procédé suivant l'une des revendications précédentes, **caractérisé en ce que** l'on produit itérativement l'image (4) de suite en trois dimensions, dans lequel, dans un stade (S4) d'itération suivant, l'image (4) de suite en trois dimensions est mise à profit comme nouvelle image (2) d'origine et, ensemble avec une nouvelle image (3) de mise à jour, on produit une nouvelle image (4) de suite en trois dimensions.

12. Procédé suivant l'une des revendications précédentes, **caractérisé en ce que** l'on se procure plusieurs images (2) d'origine et/ou **en ce que** l'on enregistre plusieurs images (3) de mise à jour, l'image (4) de suite en trois dimensions étant produite à partir de plusieurs images (2) d'origine et/ou des plusieurs images (3) de mise à jour.

13. Procédé suivant l'une des revendications précédentes, **caractérisé en ce que** l'on fait subir au réseau (5) neuronal artificiel un apprentissage, au moins en partie, à l'aide d'images (7) de test, les images (7) de test comportant des images (26) de mise à jour de fracture d'os, notamment d'examens par imagerie antérieurs et/ou des images (25) de mise à jour simulées, qui sont produites à l'aide d'une représentation en trois dimensions d'une fracture d'os.

14. Procédé suivant l'une des revendications précédentes, **caractérisé en ce que** l'on fait subir au réseau (5) neuronal artificiel un apprentissage spécifique à la région du corps à examiner.

15. Appareil (1) d'imagerie médicale pour effectuer un examen par imagerie, comprenant
- une unité (20) de mise à disposition pour se procurer une image (2) d'origine d'une région du corps ;
- une unité (21) d'enregistrement pour l'enregistrement d'une image (3) de mise à jour de la région du corps ; et
- un réseau (5) neuronal artificiel ayant subi un apprentissage conforme aux prescriptions pour la production d'une image (4) de suite en trois dimensions à partir de l'image (2) d'origine et de l'image (3) de mise à jour.
